# EUROPEAN PATENT SPECIFICATION

(11) **EP 2 311 368 B1**
(45) Date of publication and mention of the grant of the patent: **14.03.2012**
(21) Application number: 10177974.2
(22) Date of filing: 21.09.2010
(51) Int. Cl.: A61B 5/00, A61B 5/04, A61N 1/37

(54) **Orientation determination for an implantable medical device**
Ausrichtungsbestimmung für eine implantierbare medizinische Vorrichtung
Détermination d'orientation pour dispositif médical implantable

(30) Priority: 14.10.2009 US 251328 P
(43) Date of publication of application: 20.04.2011
(73) Proprietor: Biotronik CRM Patent AG, 6341 Baar (CH)
(72) Inventor: Moulder, J. Christopher, Portland, OR 97202 (US)
(74) Representative: Lindner-Vogt, Karin L.

(56) References cited:
- EP-A2- 1 995 685
- WO-A1-2006/039693
- US-A1- 2003 023 175
- US-A1- 2003 083 587

## Description

The invention relates to implantable medical devices, in particular to subcutaneous implantable medical devices capable of detecting electrical signals from the heart for monitoring heart activity.

Present day implantable heart monitoring devices use subcutaneous electrodes to sense electrical signals from the heart. This design has an increased susceptibility to noise from many sources, compared to signals detected by leads placed on the heart tissue, and is highly dependent upon orientation of the device with respect to the mean heart vector. Proper placement of the device is required during implantation to ensure optimal signal amplitude. After implantation, it is difficult to identify movement of the device from the optimal position. Such a displacement can cause decreased QRS amplitudes that affect detection algorithms or other monitoring functions of the device. Inspection of the orientation of the device by a physician requires the patient to travel to the physician and possibly receive an X-ray. Therefore there is a need for an improved method to determine the orientation of implantable heart monitoring devices.

An implantable medical device is provided, comprising an hermetically sealed, biocompatible housing and at least three subcutaneous electrodes that have fixed relative spacing for detecting electrical cardiac signals. The amplitude ratios of electrical cardiac activity for at least three combinations of two electrodes, forming at least three sensing vectors are compared with a reference to determine an orientation of the device.

Techniques for analysis and comparison of cardiac signals are disclosed in EP 1995685 A2.

The present invention will now be explained in greater detail on the basis of exemplary embodiments with reference to the figures. In the Figures
- Figure 1: shows a vector orientation with three vectors used for biopolar surface ECG recordings.
- Figure 2: shows three recording vectors I, II and III of Figure 1 and the resulting mean cardiac vector.
- Figure 3A: shows a representation of a possible electrode placement at the implantable medical device.
- Figure 3B: shows an alternative representation of a possible electrode placement at the implantable medical device.
- Figure 4A: shows the amplitude of electrical signals recorded with the electrode place- ment of Figure 3A.
- Figure 4B: shows the absolute amplitude and the sum of amplitudes of electrical signals recorded with the electrode placement of Figure 3A.
- Figure 5A: shows the amplitude of electrical signals recorded with the electrode place- ment of Figure 3B.
- Figure 5B: shows the absolute amplitude and the sum of amplitudes of electrical signals recorded with the electrode placement of Figure 3B.
- Figure 6: shows an orientation of the device to the mean cardiac vector
- Figure 7: shows amplitude ratios and sign indicator for signals recorded with the elec- trode placement of Figure 3A

Electrocardiography (ECG) is well known in the art as a transthoracic interpretation of the electrical activity of the heart detected by skin electrodes. Electrical impulses in the heart originate in the sinoatrial node and travel through the intrinsic conducting system to the heart muscle. The impulses stimulate the myocardial muscle fibers to contract and thus induce systole. The electrical waves can be measured at selectively placed electrodes (electrical contacts) on the skin. An ECG represents the voltage between pairs of these electrodes, and the muscle activity that they measure, from different directions, or vectors.

At a minimum, an ECG includes the limb leads (I, II and III). Electrodes are placed at the left and right arm and the left leg lead. I is the signal between the electrode on the right arm (negative) and the electrode on the left arm (positive). II is the signal between the electrode on the right arm (negative) and the electrode on the left leg (positive). III is the signal between the electrode on the left arm (negative) and the electrode on the left leg (positive).

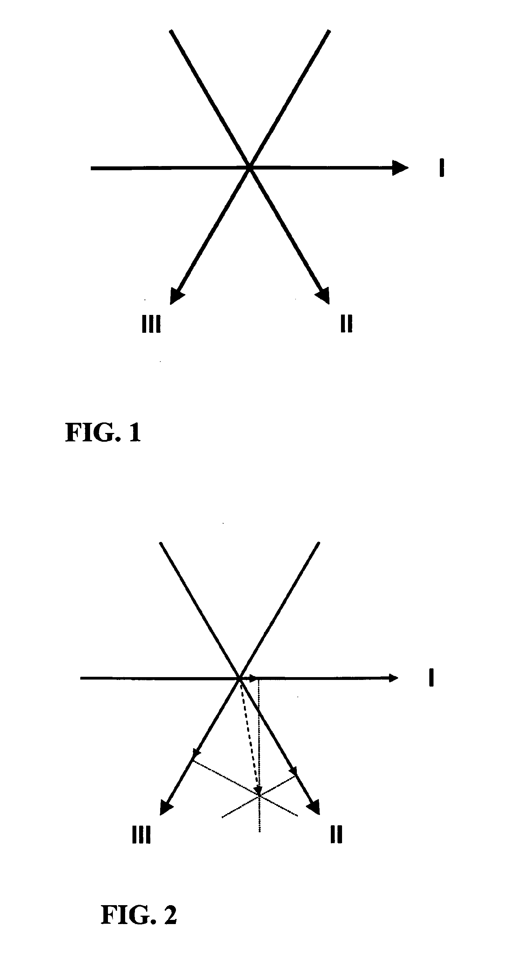

As stated above, the signals between pairs of electrodes are understood as vectors, wherein the amplitude of the vector is the voltage between the pair of electrodes and the direction (theta) of the vector is determined by the arrangement of the electrode pairs. Figure 1 shows the vectors of the limb lead signals (I, II and III) as stated above. This vector orientation is also known as Einthoven's triangle.

With the limb lead signals (I, II and III) the mean cardiac vector can be determined defined as the resultant electrical conduction vector of the three vectors (I, II and III). In Figure 2 the three recording vectors I, II and III of Figure 1 are shown, with the amplitudes at the same instant of time (e.g. at the peak of the QRS complex) depicted by the length of the arrows from the crossing point. The dashed arrow represents the direction of the resulting cardiac vector.

The implantable medical device according to this invention, referred hereafter as "device" records the electrical activity of the heart in the same fashion as described for the ECG but using at least three subcutaneous electrodes that may be placed on the housing of the device instead of electrodes placed on the surface of the body. The device is implanted subcutaneously, therefore the resultant recording is called subcutaneous electrocardiogram (SECG).

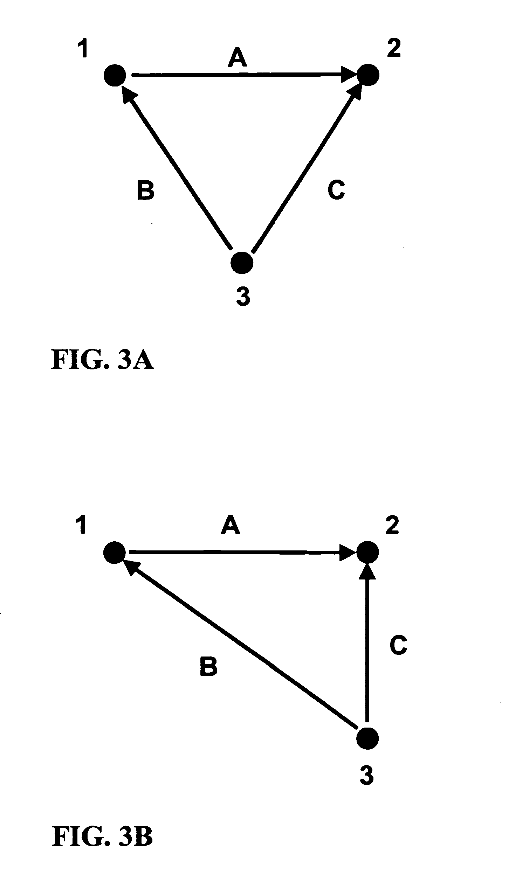

Figure 3A and Figure 3B show representations of two possible electrode placements. Electrical signals originating from cardiac activity are detected from the electrodes 1, 2 and 3 symbolised by the black circles in Figure 3A and Figure 3B. It is understood that the processing of the electrical signals detected between two electrodes is performed in the same fashion as known for e.g. pacemakers for signals detected by bipolar electrodes implanted in the heart. In particular it is understood that signals detected between the various electrode combinations described hereafter are processed simultaneously. The three electrodes allow the recording of three subcutaneous electrocardiogram (SECG) vectors in the same way as for an ECG.

Referring to Figure 3A and Figure 3B, signals detected between electrodes 1 and 2 are recorded as vector A; signals detected between electrodes 3 and 1 are recorded as vector B and signals detected between electrodes 3 and 2 are recorded as vector C. The arrows depicted as A, B and C show the orientation of the vectors. In Figure 2A the vectors A, B and C form an isosceles triangle very similar to Einthoven's triangle as shown in Figure 1. In Figure 3B the vectors A, B and C form a scalene right triangle wherein with two of the three recording vectors are orthogonal.

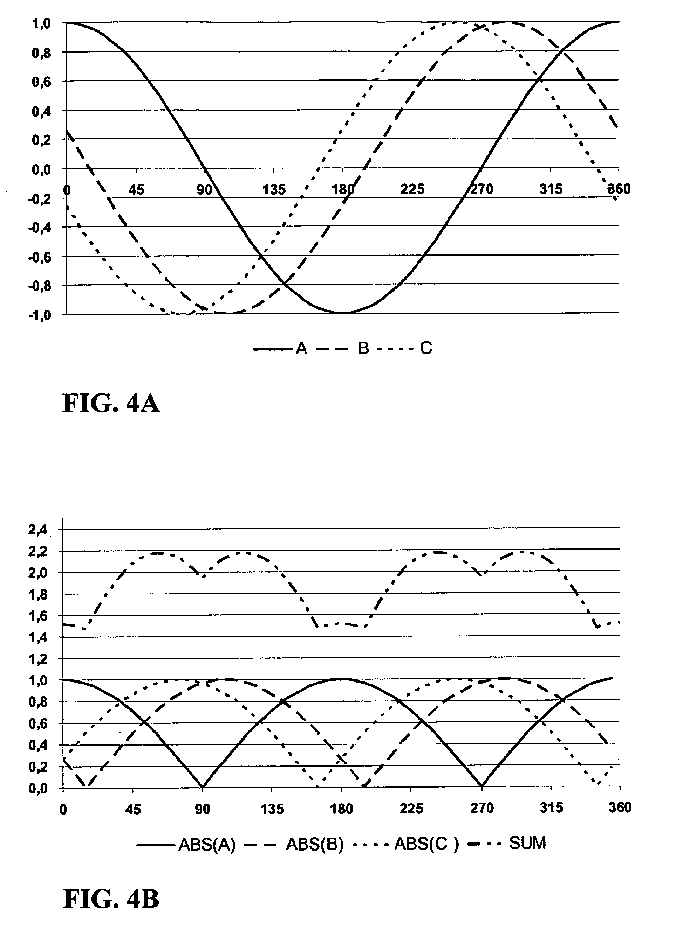

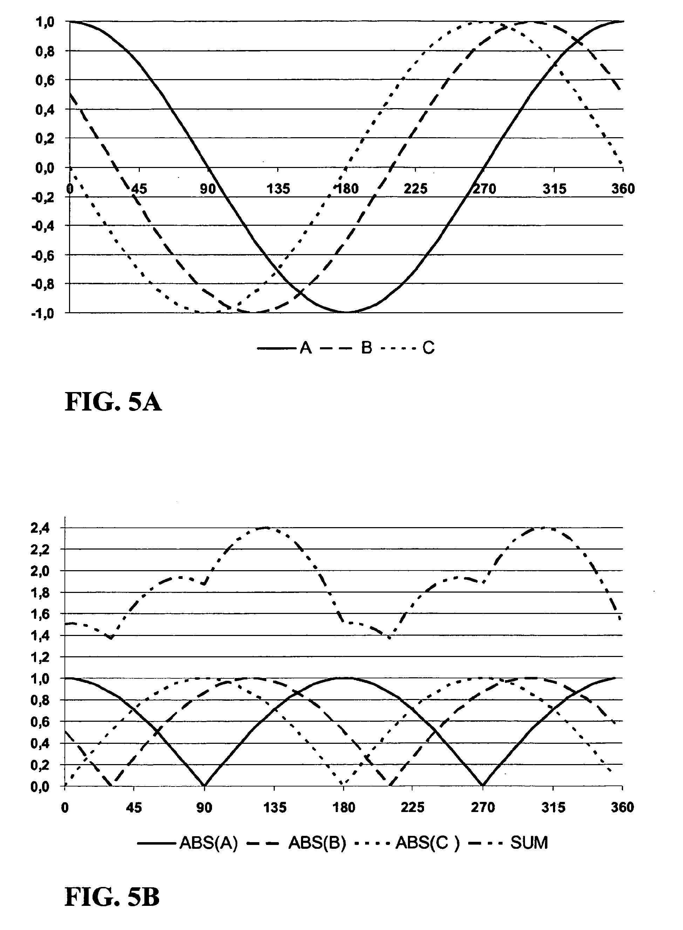

It can be seen that the amplitude of each vector is dependent upon the angle of the vector with respect to the orientation to the heart. Figure 4A shows the amplitude relationship for the electrode configuration shown in Figure 3A and Figure 5A shows the amplitude relationship for the electrode configuration shown in Figure 3B. The orientation of the trianglular electrode configuration is rotated relative to the heart between 0 degree and 360 degree. The abscissa in Figure 4A and Figure 5A shows the orientation of vector A relative to the cardiac vector in degree is shown, the ordinate represents the normalized amplitude of the vectors A, B and C.

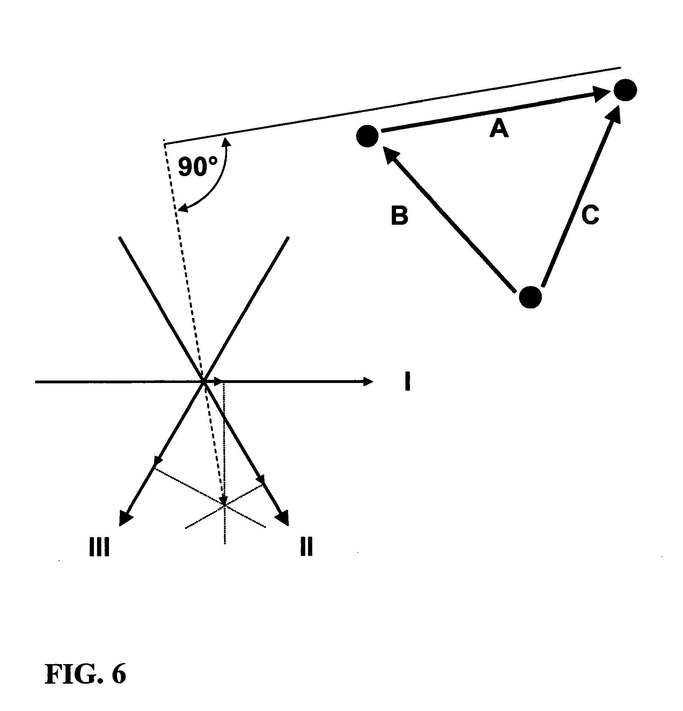

Figure 6 shows, as one example, an orientation of the device with the electrode configuration as shown in Figure 3A to the cardiac vector as shown in figure 2. In this example, the orientation of vector A is 90 degree relative to the mean heart vector.

The optimal orientation of the device relative to the mean heart vector can be determined by summing up the absolute values of each vector amplitude. This is shown in Figure 4B for the electrode configuration shown in Figure 3A and in Figure 5B for the electrode configuration shown in Figure 3B. The absolute amplitudes of the vectors A, B and C are plotted as solid, dashed and dotted line, the sum is plotted as dashed-dotted line above.

The optimal orientation of the device can be determined as an orientation relative to the max heart vector where the sum exceeds a predetermined threshold.

The amplitudes of the vectors A, B and C recorded at the same point of time depend on the amplitude of cardiac activity and the distance of the device to the heart. However the amplitude ratios of the vectors remain constant and depend only on the orientation of the device relative to the heart vector.

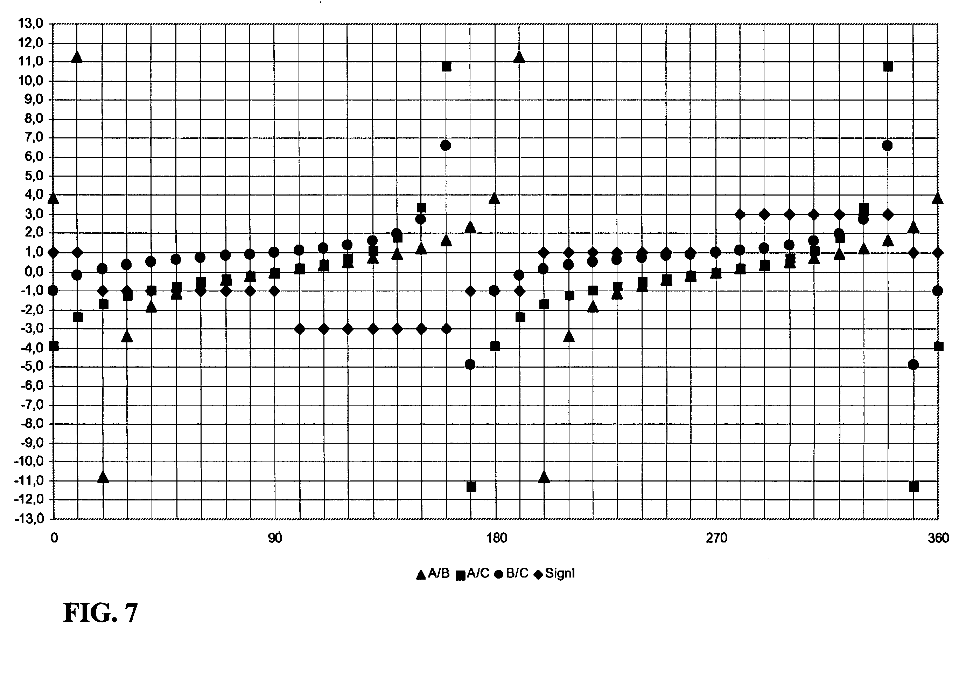

In order to determine the orientation of the device relative to the mean heart vector the amplitude ratios of the vectors are evaluated. Figure 7 shows the amplitude ratios of the vectors shown in Figure 4A. Again the x-axis in represents the orientation of vector A relative to the cardiac vector in degrees. Plotted are the ratios A/B as triangles, the ratio A/C as quadrates and the ratio B/C as circles in steps of 10 degree.

It can be seen that the combination of the three amplitude ratios shown [A/B, A/C, B/C] is unique for each orientation of the cardiac device relative to the mean heart vector for a range of 0 degree to 180 degree. If, in addition to the amplitude ratios, the signs of the input signals are evaluated, a unique combination for each orientation of the device relative to the mean heart vector can be found. The evaluation of signs can be done by converting the signs of the vectors into integer values (positive sign equals 1, negative sign equals -1 and 0 otherwise) and sum these integer values afterwards. Such a sign indicators are denoted as SignI in Figure 7 and plotted as rhombuses. Therefore, a data set representing an orientation of the device relative to the heart vector comprises for particular orientation, the corresponding three amplitude ratios [A/B, A/C, B/C] and the sign indicator as elements and is hereafter referred to as orientation data set.

Alternatively, the orientation data set may be derived from the ratio of A:B:C as unique identifier of the orientation.

Orientation data sets can be stored as references for known orientations of the device relative to the heart vector. These reference Orientation data sets can be determined in different ways. As the orientations of the vectors A, B and C relative to each other are determined by electrode orientation, expected vector ratios and sign indicator can be calculated for different orientations and stored as reference orientation data sets. Alternatively the device can placed in a fluid with similar attributes as the body fluid, e.g. in a water bath, and vector ratios and sign indicator for different orientations relative to a known signal vector representing the peak mean heart vector can be recorded and stored as reference orientation data sets.

The determination of the orientation of the device relative to the mean heart vector can now be performed by comparing actual orientation data sets with the stored reference orientation data sets. The comparison can be performed in various ways, e.g. by storing reference orientation data sets as look-up table and seeking for a 1 to 1 match or the closest match of the orientation data set elements.

It is also possible to determine changes of the orientation change of the device. In this case, the orientation data set at a first point of time is stored as reference. In the event of gross changes in the SECG, a new comparison can be performed to determine if the device changed position. This determination does not need any external devices and thus can be performed remotely.

### Description of embodiments

In the preferred embodiment the implantable medical device comprises at least three electrodes that are placed at or on the hermetically sealed, biocompatible housing for detecting electrical cardiac signals. An electrode configuration is shown in Figure 3A. An alternative electrode configuration is shown in Figure 3B.

In the preferred embodiment, sensing means are coupled to the electrodes for sensing cardiac activity in at least three vectors. The sensing means measures the amplitude of electrical signals between two electrodes. Suitable sensing means are known in the art such as sensing means implemented in implantable loop recorders, pacemakers or defibrillators. The sensing means can further comprise one or more of sense amplifier, filter and analog/digital converter. The sensing means can be embodied to sample the input signals with a predetermined sampling rate.

For the electrode configuration shown in Figure 3A, the sensing means measures the amplitude of signals detected between electrodes 1 and 2 as vector A. The amplitude of signals detected between electrodes 3 and 1 as is measured as vector B and the amplitude of signals detected between electrodes 3 and 2 is measured as vector C. The measurements are performed simultaneously. The sensed amplitudes may be stored in the memory.

In the preferred embodiment, processing means are coupled to the sensing means to process the measured vectors. Suitable processing means are known in the art such as processing means implemented in implantable loop recorders, pacemakers or defibrillators. The processing means may for example be implemented in the form of a programmable microcontroller to perform the required processing operations.

In the preferred embodiment the processing means are coupled to memory means.

In the preferred embodiment, telemetry means are coupled to the processing means and/or to the memory means to transmit data recorded by the implant or stored in the memory of the implant to an external device. Suitable telemetry means are known in the art such as inductive or RF based telemetry means implemented in implantable loop recorders, pacemakers or defibrillators. The external device may be further wired or wireless connected to a remote server, expert centre or database.

In the preferred embodiment which does not form part of the invention, the processing means detects the QRS-complex in the sensed vector signals and provides the amplitudes of the sensed vector signals at the point of time where the QRS complex occurs.

In a further embodiment which does not form part of the invention, the processing means averages the sensed vector signals and detects the QRS complex in the averaged signal.

In a further embodiment which does not form part of the invention, the processing means verifies whether the maximum amplitudes of the sensing vectors occur at the same point of time and provide a corresponding indicator.

In the preferred embodiment, the processing means calculates the amplitude ratios of the sensed vector signals. In a further embodiment the calculated amplitude ratios are stored in the memory means.

In the preferred embodiment, the processing means calculates a sign indicator by converting the signs of amplitude of the input vector signals into integer values, wherein positive sign equals 1, negative sign equals -1 and 0 otherwise, and summing these integer values. In a further embodiment, the calculated sign indicator is stored in the memory means.

In the preferred embodiment, the processing means generates and/or stores a orientation data set comprising the amplitude ratios and sign indicator of the amplitudes of the sensed vector signals of the same point of time.

In the preferred embodiment reference at to least one reference orientation data set comprising a orientation data set and the orientation relative to the mean heart vector is stored. In a further embodiment which does not form part of the invention, reference orientation data sets are stored as a look-up table.

In the preferred embodiment which does not form part of the invention, the processing means determines the device orientation relative to the maximum or mean heart vector by comparing the amplitude ratios and sign indicator of one orientation data set with the amplitude ratios of one or more reference orientation data sets and determine the orientation of vector A relative to the heart vector of the reference data set where the amplitude ratios and sign indicator are identical.

In a further embodiment which does not form part of the invention the processing means determines the orientation relative to the heart vector of the reference data set where the amplitude ratios and sign indicator are within a predefined range.

## Claims

1. An implantable medical device, comprising: a hermetically sealed, biocompatible housing; at least three subcutaneous electrodes connected to the device for detecting electrical cardiac signals; sensing means connected to said electrodes for sensing amplitudes of electrical cardiac activity for at least three combinations of two electrodes, forming at least three sensing vectors; memory means; telemetry means and processing means connected to said sensing means, memory means and telemetry means, **characterised in that** said processing means being implemented to determine amplitude ratios of said at least three sensing vectors; wherein said memory means is implemented to store said amplitude ratios for at least one orientation of the device and wherein said processing means is implemented to determine an orientation of the device by comparing said determined amplitude ratios to stored amplitude ratios.

2. The device of claim 1, wherein the processing means is further implemented to determine a sign indicator for said amplitudes and wherein the memory means is further implemented to store said sign indicator for at least one orientation of the device and wherein the processing means is further implemented to determine an orientation of the device by comparing said determined amplitude ratios to stored amplitude ratios and by comparing said determined sign indicator to stored sign indicators.

3. The device of claim 1, wherein said memory means stores the time of said stored orientation.

4. The device of claim 1, wherein said memory means stores said determined orientation.

5. The device of claim 4, wherein said memory means stores the time of said determined orientation.

6. The device of claim 1 wherein one or all of amplitudes, amplitude ratios and orientation are transmitted by the telemetry means.

7. The device of claim 2 wherein one or all of amplitudes, amplitude ratios, sign indicator and orientation are transmitted by the telemetry means.

8. The device of claim 1, wherein the amplitude ratios are determined at the time of occurrence of the QRS complex.

## Patentansprüche

1. Implantierbares Medizinprodukt, umfassend: ein hermetisch abgedichtetes, biokompatibles Gehäuse, mindestens drei subkutane Elektroden, die mit dem Produkt verbunden sind, um elektrische Herzsignale zu erfassen, Sensormittel, die mit den Elektroden verbunden sind, um Amplituden der elektrischen Herzaktivität bei mindestens drei Kombinationen von zwei Elektroden zu erfassen, die mindestens drei Erfassungsvektoren bilden, Speichermittel, Fernmessmittel und Verarbeitungsmittel, die mit den Erfassungsmitteln, Speichermitteln und Fernmessmitteln verbunden sind, **dadurch gekennzeichnet, dass** die Verarbeitungsmittel vorgesehen sind, um Amplitudenverhältnisse der mindestens drei Erfassungsvektoren zu ermitteln, wobei das Speichermittel vorgesehen ist, um die Amplitudenverhältnisse für mindestens eine Ausrichtung des Produkts zu speichern und wobei das Verarbeitungsmittel vorgesehen ist, um eine Ausrichtung des Produkts durch Vergleichen der ermittelten Amplitudenverhältnisse mit gespeicherten Amplitudenverhältnissen zu ermitteln.

2. Produkt nach Anspruch 1, wobei das Verarbeitungsmittel ferner vorgesehen ist, um einen Vorzeichenanzeiger für die Amplituden zu ermitteln und wobei das Speichermittel ferner vorgesehen ist, den Vorzeichenanzeiger für mindestens eine Ausrichtung des Produkts zu speichern und wobei das Verarbeitungsmittel ferner vorgesehen ist, eine Ausrichtung des Produkts durch Vergleichen der ermittelten Amplitudenverhältnisse mit gespeicherten Amplitudenverhältnissen und durch Vergleichen des ermittelten Vorzeichenanzeigers mit gespeicherten Vorzeichenanzeigern zu ermitteln.

3. Produkt nach Anspruch 1, wobei das Speichermittel den Zeitpunkt der gespeicherten Ausrichtung speichert.

4. Produkt nach Anspruch 1, wobei das Speichermittel die ermittelte Ausrichtung speichert.

5. Produkt nach Anspruch 4, wobei das Speichermittel den Zeitpunkt der ermittelten Ausrichtung speichert.

6. Produkt nach Anspruch 1, wobei von den Fernmessmitteln ein(e) oder alle Amplitude(n), Amplitudenverhältnis(se) und Ausrichtung(en) übertragen werden.

7. Produkt nach Anspruch 2, wobei von den Fernmessmitteln ein(e) oder alle Amplitude(n), Amplitudenverhältnis(se), Vorzeichenanzeiger und Ausrichtung(en) übertragen werden.

8. Produkt nach Anspruch 1, wobei die Amplitudenverhältnisse zum Zeitpunkt des Auftretens des QRS-Komplexes ermittelt werden.

## Revendications

1. Dispositif médical implantable, comprenant : un boîtier biocompatible fermé hermétiquement ; au moins trois électrodes sous-cutanées reliées au dispositif pour la détection de signaux cardiaques électriques ; un moyen de détection relié auxdites électrodes pour la détection des amplitudes de l'activité cardiaque électrique, pour au moins trois combinaisons de deux électrodes, formant au moins trois vecteurs de détection ; une mémoire ; un moyen de télémétrie et un moyen de traitement relié audit moyen de détection, à ladite mémoire et audit moyen de télémétrie, **caractérisé en ce que** ledit moyen de traitement est mis en oeuvre pour déterminer les ratios d'amplitude desdits au moins trois vecteurs de détection ; et ladite mémoire est mise en oeuvre pour stocker lesdits ratios d'amplitude, pour au moins une orientation du dispositif, en comparant lesdits ratios d'amplitude déterminés avec des ratios d'amplitude stockés.

2. Dispositif selon la revendication 1, étant le moyen de traitement en outre mis en oeuvre pour déterminer un indicateur de signe pour lesdites amplitudes, et la mémoire étant en outre mise en oeuvre pour stocker ledit indicateur de signe pour au moins une orientation du dispositif, et le moyen de traitement étant en outre mis en oeuvre pour déterminer une orientation du dispositif, en comparant lesdits ratios d'amplitude déterminés avec des ratios d'amplitude stockés, et en comparant ledit indicateur de signe déterminé avec des indicateurs de signe stockés.

3. Dispositif selon la revendication 1, dans lequel ladite mémoire stocke le temps de ladite orientation stockée.

4. Dispositif selon la revendication 1, dans lequel ladite mémoire stocke ladite orientation déterminée.

5. Dispositif selon la revendication 4, dans lequel ladite mémoire stocke le temps de ladite orientation déterminée.

6. Dispositif selon la revendication 1, dans lequel une ou toutes les amplitudes, ratios d'amplitude et orientations sont transmis par le moyen de télémétrie.

7. Dispositif selon la revendication 2, dans lequel une ou toutes les amplitudes, ratios d'amplitude, indicateurs de signe et orientations sont transmis par le moyen de télémétrie.

8. Dispositif selon la revendication 1, dans lequel les ratios d'amplitude sont déterminés au moment de survenue du complexe QRS.
